# EUROPEAN PATENT APPLICATION

(11) **EP 1 371 425 A1**
(43) Date of publication of application: **17.12.2003**
(21) Application number: 03076775.0
(22) Date of filing: 06.06.2003
(51) Int. Cl.: B07C 5/10, B07C 5/342, G01N 33/02

(54) **Detector**

(30) Priority: 13.06.2002 EP 02077281
(71) Applicant: FPS Food Processing Systems B.V., 2631 RE Nootdorp (NL)
(72) Inventor: Crezee, Leonardus Paulus, 3425 ET Snelrewaard (NL)
(74) Representative: Prins, Adrianus Willem

(57) **Abstract**

An apparatus for determining the light transmission characteristic of an object, having arranged near the object,
- a light source for illuminating at least a first part of the surface of the object with a light beam,
- a screening device for screening at least a second part of the surface from the light beam, and
- a light receiver for receiving light coming from at least a part of the second part of the surface after transmission by the object,
characterized in that the second part is at least partly contiguous to the first part, while preferably the second part is wholly enclosed by the first part.

In an advantageous exemplary embodiment, the screening device is movable relative to at least the light receiver.

Such an apparatus is highly suitable to be used in a sorting apparatus for selecting and sorting a continuous stream of objects, such as fruits, according to light transmission characteristic.

## Description

The present invention relates to an apparatus as indicated in the preamble of claim 1.

Such an apparatus is known from US 5,089,701, which discloses a device by means of which, in a nondestructive manner, upon transit of light from the near-infrared wavelength region, a transmission spectrum can be obtained for the determination of, for instance, the sugar content in fruits. To accurately determine how much light enters the fruit and subsequently exits the fruit again at a particular point, while it has moreover been ensured that the transmission path is sufficiently long, tubular light guiding nozzles are placed on the surface of the fruit at the location of entry position and exit position, while moreover a well-defined minimum distance between these positions has been chosen. A somewhat similar measuring arrangement is known from Kawano *et al.,* "Determination of Sugar Content in Intact Peaches by NIR Spectroscopy with Fiber Optics in Interactance Mode", J. Japan. Soc. Hort. Sci., 61(2), 445-451, 1992, where optical fibers are applied having, viewed in cross section, a central exit fiber portion which is surrounded and enclosed by an annular entry fiber portion, with entry/exit for directing the light of the light beam towards and away from the fruit, respectively.

Clearly, neither apparatus is suitable for online measurements, that is, measurements during transit on a sorting machine. It is precisely such measuring conditions that impose stringent requirements on a detector, in particular the combination of speed, accuracy, and repeatability.

What US 5,089,701 contemplates is to bring about intermediate distances, which enables the light path, under the limitation of the two positions mentioned, to be chosen sufficiently large. In Kawano *et al.,* a very compact arrangement for a desktop probe is represented, where not only a transit beam of the transmission light will enter the exit end, but also reflection light. It has been found that this reflection light provides entirely different information than the information sought in the case of transmission.

In order to remedy the shortcomings mentioned, the present invention provides an apparatus as mentioned above, characterized in that the second part mentioned is at least partly contiguous to the first part.

In this way, a highly advantageous compact arrangement is obtained, at the same time featuring a screening from reflection light.

In a further exemplary embodiment, this apparatus is characterized in that the second part mentioned in wholly enclosed by the first part.

A particular advantage of this arrangement is that further a very well settable intensity of entering light can be set that is adjustable and repeatable for each type of fruit.

Yet another exemplary embodiment of the apparatus according to the present invention is characterized in that at least the screening device and the light receiver are accommodated in the same holder, in particular that the screening device is moveable relative to at least the light receiver; furthermore, that the screening device consists of a screening cap arranged on a tubular holder, and more in particular that the light source is arranged on the holder and is fixedly positioned relative to the light detector.

In addition, the present invention comprises a sorting apparatus for selecting and sorting a continuous stream of objects, such as fruits, according to light transmission characteristic, characterized by the apparatus having any one of the above-mentioned features.

Such a sorting apparatus enables highly accurate and reliable sorting according to sugar content.

Further details of the present invention will be elucidated with reference to a drawing, in which
Fig. 1 schematically represents the invention, and
Fig. 2 shows an exemplary embodiment of the invention in perspective.

In the two figures, the same parts have been given the same reference numerals.

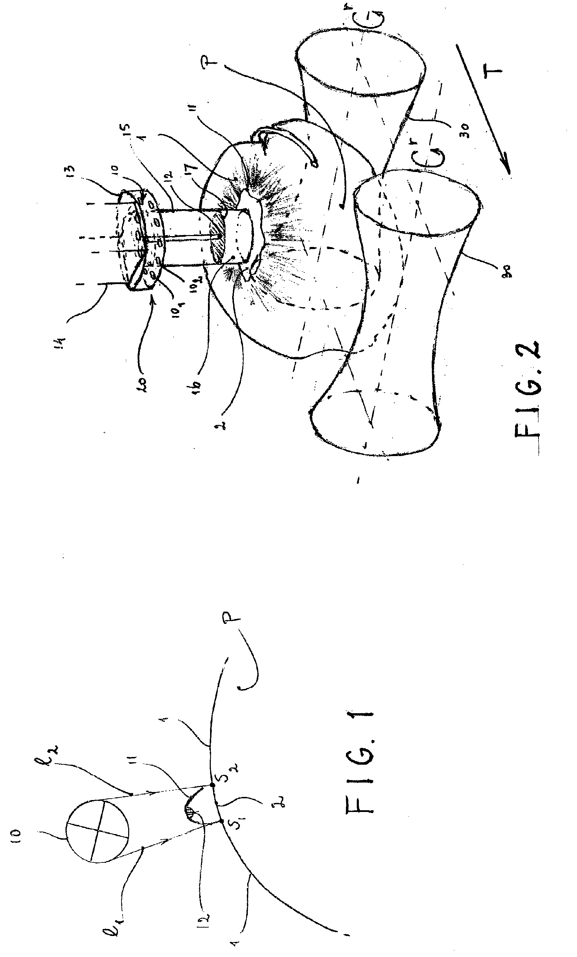

Fig. 1 shows, in a cross-sectional view, a product P such as, for instance, a fruit, more particularly an apple. With a light source 10, P is illuminated, more particularly only there where the product is not screened from the light source 10 with a screening 11. Represented is the situation where the screening does not rest on the product P but is positioned at a slight distance therefrom. Depending on the measuring conditions, it is possible to opt for the measuring setup without contact, as represented, or for the other measuring setup, with contact, merely mentioned above. For the type of measurements through which the sugar content in the interior of fruits can be determined, the light source will be capable of emitting light in the wavelength range between 300 nm and 1500 nm. In addition, suitably, if necessary, light signal variations can be used, for instance pulsed light. In the figure, it can be seen that in this arrangement a first part 1 of the surface is illuminated, and there the light can penetrate the product P, and that a second surface part 2 is formed between S1 and S2, which is wholly screened from the light source 10. To illustrate this, two light rays 11 and 12 are schematically represented in the figure, which define the boundary or the edge of the non-illuminated surface part 2. In other words, as a result of the position of the screening 11 with respect to the light source 10, a shaded area S1S2 is obtained. The first part 1 of the surface, located outside S1S2, will be illuminated to a partial or full extent, depending on the construction of the light source. The light entering by way of the first part 1 has the possibility of leaving the product P again by way of the second part 2, depending on the optical properties of the product. This exiting light is then received in a light receiver 12. Further guidance and processing of this light will not be elucidated in detail here. However, it will be clear to anyone skilled in the art that fiber optics can be applied advantageously, while the processing of the light signals for determining, for instance, transmission characteristics of such products can be carried out with a computer.

In Fig. 2, an exemplary embodiment of such a setup is shown in perspective view. More particularly, a product P, for instance an apple, rests on diabolo-shaped rollers 30 of a sorting machine generally known in the art, not shown here. The machine can be so arranged that the rollers 30 can rotate about their cylinder symmetry axis, indicated by arrows r, and hence the products P can rotate as well. In general, the products to be tested will pass a detection unit, here indicated with an arrow T for the transport direction. At such a unit, a measuring head 20 must be positioned near or against a product for it to be tested or measured.

In the exemplary embodiment shown, the measuring head 20 is a holder for both the light source 10 and the screening 11. More in particular, light sources 101, 102, ... are arranged in an annular holder 13. These are, for instance, a number of light emitting diodes, or LEDs, or halogen light sources having well-focused beams to thereby obtain sufficient beam intensity. By way of a coupling piece 14, the measuring head is connected with the larger whole of the detection unit mentioned, with all signal connections passing via this coupling piece. Further, this coupling piece 14 accommodates the positioning mechanism, that is, means for moving the measuring head towards the product and away from the product. On the other side of the annular holder 13, a tube 15 is arranged, in which the light receiver 12 is disposed. In this exemplary embodiment, an optic fiber is used. The connection and guidance thereof are also arranged in the coupling piece 14. Adjacent the fiber end, a closing cap 17 is arranged in the tube 15, thereby forming a tube end 16. This tube end is so designed that the light that finds its way there ends up at the fiber end as best as possible to be able to contribute to the signal to be processed. For instance, surfaces of proper curvature and mirror properties are used, or a system with one or more lenses, or a combination thereof. Arranged at this fiber end is the screening 11, to be regarded as a flap of suitable plastic, to be able to conform to the shape of the product and thereby afford proper screening. In a different embodiment, a metal cap of suitable, and possibly even variable, dimensions is used, which, though not flexible, can yet be placed close to or against the fruit and is better resistant to the heat of the lamps. In this figure also, the first part 1 and the second part 2 of the surface of the product P are indicated. The first part 1, being illuminated, has been hatched. Further, it will be clear that, depending on curvature of the product and positioning of the light sources 101, 102, .. the screening must rest on the product or may hang a slight distance above it.

In a sorting machine, several of such measuring heads 20 may be arranged behind each other to measure the same fruit a corresponding number of times in order to arrive at measuring results with a smaller margin of error, while as a result of the rotating rollers, in each case the detector is placed on a different part of the fruit. Furthermore, a unit can have several of such rows of measuring heads next to each other, to thereby enable testing of rows of products passing next to each other over the same conveyor.

In an exemplary embodiment not shown in a figure, an arrangement has been chosen where only the screening 11, for instance designed as a tube, can be moved. Preferably, this movement involves telescopic extension and retraction. What is thus achieved is that, by contrast, the optical components, viz. the light sources and the optical fibers, do not need to be moved. Thus, wear, small changes in the relative positioning, and wire and/or fiber rupture are advantageously avoided.

Furthermore, it is observed that the detector possesses a fixed position with respect to the sorting machine. In most cases, this involves a mounting frame that is fixedly connected with this machine.

It will be clear from the foregoing that because of the compact design, with great advantage a measuring head is provided that can be fitted into many types of sorting machines. Moreover, this enables online testing, often automatic and rapid.

It will be clear to anyone skilled in the art that for the specific choice of components there are several options, especially where the light sources, light guidance and light focusing are concerned. Furthermore, in this field of the art, it is known how such signals must be processed, in particular for determining characteristics, and how to take these into account in the selection of the products, intended for the actuation of ejection or delivery means. Further small modifications are to be regarded as falling within the appended claims.

## Claims

1. An apparatus for determining the light transmission characteristic of an object, having arranged near the object:
- a light source for illuminating at least a first part of the surface of the object with a light beam,
- a screening device for screening at least a second part of the surface from said light beam, and
- a light receiver for receiving light coming from at least a part of said second part of the surface after transmission by the object,
**characterized in that**
said second part is at least partly contiguous to the first part.

2. An apparatus according to claim 1, **characterized in that** said second part is wholly enclosed by the first part.

3. An apparatus according to claim 1 or 2, **characterized in that**
at least the screening device and the light receiver are accommodated in the same holder.

4. An apparatus according to claim 3, **characterized in that**
the screening device is movable relative to at least the light receiver.

5. An apparatus according to claim 3 or 4, **characterized in that**
the screening device consists of a screening cap arranged on a tubular holder.

6. An apparatus according to claim 3, 4 or 5, **characterized in that** the light source is arranged on the holder and is fixedly positioned with respect to the light receiver.

7. An apparatus according to any one of the preceding claims, **characterized in that**
it possesses a fixed position with respect to a sorting machine intended for sorting the objects or products to be examined.

8. An apparatus according to claim 7, **characterized in that**
the apparatus forms part of a mounting frame.

9. An apparatus according to any one of the preceding claims, **characterized in that** the light source emits light in the wavelength range between 300 nm and 1500 nm.

10. A sorting apparatus for selecting and sorting a continuous stream of objects, such as fruits, according to light transmission characteristic, **characterized by** the apparatus according to any one of the preceding claims.
